# EUROPEAN PATENT SPECIFICATION

(11) **EP 4 332 662 B1**
(45) Date of publication and mention of the grant of the patent: **14.10.2026**
(21) Application number: 21938564.8
(22) Date of filing: 30.04.2021
(51) Int. Cl.: G02B 27/10, G01N 21/64, G02B 27/58, G02B 5/04, G02B 21/36, C12Q 1/6837, C12Q 1/6869

(54) **FIELD-OF-VIEW STITCHING SYSTEM AND METHOD, AND BIOLOGICAL SAMPLE IDENTIFICATION DEVICE AND METHOD**
SICHTFELD-STITCHING-SYSTEM UND -VERFAHREN SOWIE VORRICHTUNG UND VERFAHREN ZUR IDENTIFIZIERUNG BIOLOGISCHER PROBEN
SYSTÈME ET PROCÉDÉ DE COUTURE DE CHAMP DE VISION, ET DISPOSITIF ET PROCÉDÉ D'IDENTIFICATION D'ÉCHANTILLON BIOLOGIQUE

(43) Date of publication of application: 06.03.2024
(73) Proprietor: MGI Tech Co., Ltd., Shenzhen, Guangdong 518083 (CN)
(72) Inventor: BO, En, Shenzhen, Guangdong 518083 (CN); SONG, Liyan, Shenzhen, Guangdong 518083 (CN); LIU, Ang, Shenzhen, Guangdong 518083 (CN); LONG, Qingshan, Shenzhen, Guangdong 518083 (CN); ZHOU, Shengyuan, Shenzhen, Guangdong 518083 (CN)
(74) Representative: Zaboliene, Reda
(86) International application number: PCT/CN2021/091769
(87) International publication number: WO 2022/227091

(56) References cited:
- WO-A1-2007/088529
- CN-A- 102 692 702
- CN-A- 108 845 411
- CN-B- 106 322 301
- JP-A- 2011 180 592
- JP-A- 2016 170 302
- US-A1- 2010 073 757
- US-A1- 2015 004 637
- US-A1- 2019 129 278

## Description

### TECHNICAL FIELD

The present disclosure generally relates to optical imaging technology field, particularly relates to a field of view stitching system, a field of view stitching method applied to the field of view stitching system, a biological sample identification device based on super-resolution imaging using the field of view stitching system, and a biological sample identification method based on super-resolution imaging using the biological sample identification device which is based on super-resolution imaging.

### BACKGROUND

Gene sequencing refers to analyzing an arrangement sequence of four kinds of bases in a nucleic acid sample. With the rapid development of gene sequencing, super-resolution imaging sequencing systems have emerged for gene sequencing. The super-resolution sequencing system relies on a digital micromirror device (DMD).

The DMD is used to receive illumination light and modulate structured light to project the structured light onto a sequencing chip loaded with nucleic acid samples, which forms a specific structured illumination on a surface of the nucleic acid samples. The super-resolution imaging sequencing system can increase the arrangement density of the nucleic acid samples to be tested each time and can control spaces between nucleic acid samples to be reduced to below 200 nm. The DMD includes a plurality of micro mirrors, and areas of the plurality of micro mirrors determine an array size of the DMD. Due to limitations of the manufacture process of the DMD, the number of the micro mirrors of the DMD and the array size of the DMD both are limited. A traditional super-resolution imaging sequencing system based on the DMD is limited by the array size and the number of the micro mirrors of the DMD, which makes it difficult to expand a field of view of the structured illumination on the nucleic acid samples, and further limits a further improvement of a sequencing throughput.

US2015004637A1 discloses an illumination system for a microscope based on a digital micro-mirror device.

### SUMMARY

A first aspect of the present disclosure provides a field of view stitching system, including:
a light source module for emitting a first beam;
a light guiding element on an optical path of the first beam and configured to split the first beam into a plurality of second beams, wherein the light guiding element includes an incident surface positioned corresponding to the light source module, a first exit surface facing and spaced apart from the incident surface, and a plurality of second exit surfaces bending and extending from edges of the first exit surface towards the incident surface, the plurality of second beams exit from the first exit surface and the plurality of second exit surfaces and propagate along the optical path; and
a light modulation module comprising a plurality of light modulators, wherein, a number of the plurality of light modulators is the same as a total number of the first exit surface and the plurality of second exit surfaces, each of the plurality of light modulators is configured to receive and regularly reflect one of the plurality of second beams, each of the plurality of light modulators has an effective light modulation area, each of the plurality of second beams covers the effective light modulation area of one of the light modulators when incident on the light modulator;
wherein the light guiding element is further configured for receiving and combining the plurality of second beams reflected by the plurality of light modulators to form an illuminating light to further exit through the incident surface.

A second aspect of the present disclosure provides a biological sample identification device based on super-resolution imaging, including:
a field of view stitching system, including:
   a light source module for emitting a first beam;
   a light guiding element on an optical path of the first beam and configured to split the first beam into a plurality of second beams, wherein the light guiding element includes an incident surface positioned corresponding to the light source module, a first exit surface facing and spaced apart from the incident surface, and a plurality of second exit surfaces bending and extending from edges of the first exit surface towards the incident surface, the plurality of second beams exit from the first exit surface and the plurality of second exit surfaces and propagate along the optical path; and
   a light modulation module including a plurality of light modulators, wherein, a number of the plurality of light modulators is the same as a total number of the first exit surface and the plurality of second exit surfaces, each of the plurality of light modulators is configured to receive and regularly reflect one of the plurality of second beams, each of the plurality of light modulators has an effective light modulation area, each of the plurality of second beams covers the effective light modulation area of one of the light modulators when incident on the light modulator; wherein the light guiding element is further configured to receive and combine the plurality of second beams reflected by the plurality of light modulators to form an illuminating light to further exit through the incident surface and further configured to irradiate a biological sample to cause a detection light ;
an imaging module for receiving the detection light and generating a detection image based on the detection light; and
a control portion electrically connected to the light source module, the plurality of light modulators, and the imaging module, the control portion is configured to control an operation process of the light source module, the plurality of light modulators, and the imaging module, and to identify a biological feature of the biological sample based on the detection image.

A third aspect of the present disclosure provides a field of view stitching method applied to a field of view stitching system, wherein the field of view stitching system includes a light source module, a light guiding element, and a light modulation module, the light modulation module is provided with a plurality of light modulators, the light guiding element is provided with an incident surface, a first exit surface facing the incident surface, and a plurality of second exit surfaces bending and extending from edges of the first exit surface towards the incident surface, a number of the plurality of light modulators is the same as a total number of the first exit surface and the plurality of second exit surfaces, and each of the plurality of light modulators has an effective light modulation area; the field of view stitching method comprises:
emitting a first beam towards the incident surface by the light source module;
splitting the first beam into a plurality of second beams by the first exit surface and the plurality of second exit surfaces, and further directing the plurality of second beams to exit from the first exit surface and the plurality of second exit surfaces and propagate along an optical path of the first beam;
transmitting each of the plurality of second beams to one of the plurality of light modulators, controlling each of the plurality of light modulators to receive and regularly reflect one of the plurality of second beams, with each of the plurality of second beams covering the effective light modulation area of the light modulator; and
forming an illumination light on the incident surface by a combination of the plurality of second beams which are regularly reflected to the light guiding element along an opposite direction of the optical path and pass through the first exit surface and the plurality of second exit surfaces.

A fourth aspect of the present disclosure provides a biological sample identification method based on super-resolution imaging and is applied to a biological sample identification device, the biological sample identification device is provided with a field of view stitching system including a light source module, a light guiding element, and a light modulation module, the light modulation module includes a plurality of light modulators, the light guiding element is provided with an incident surface positioned corresponding to the light source module, a first exit surface facing and positioned apart from the incident surface, and a plurality of second exit surfaces bending and extending from edges of the first exit surface towards the incident surface, a number of the plurality of light modulators is the same as a total number of the first exit surface and the plurality of second exit surfaces, and each of the plurality of light modulators has an effective light modulation area; the biological sample identification method based on super-resolution imaging comprises:
emitting a first beam towards the incident surface by the light source module;
splitting the first beam into a plurality of second beams by the first exit surface and the plurality of second exit surfaces, and further directing the plurality of second beams to exit from the first exit surface and propagate along an optical path of the first beam;
transmitting each of the plurality of second beams to one of the plurality of light modulators, controlling each of the plurality of light modulators to receive and regularly reflect one of the plurality of second beams, with each of the plurality of second beams covering the effective light modulation area of the light modulator;
forming an illumination light on the incident surface by a combination of the plurality of second beams which are regularly reflected to the light guiding element along an opposite direction of the optical path and pass through the first exit surface and the plurality of second exit surfaces;
irradiating a biological sample with the illumination light through the incident surface to generate a detection light from the biological sample;
collecting the detection light and generating a detection image based on the detection light; and
identifying a biological feature of the biological sample based on the detection image.

The field of view stitching system can be applied to various types of optical system. The light guiding element includes the first exit surface and the second exit surface. The light guiding element is configured to split the first beam emitted by the light source module, which makes the first exit surface transmits the second beams, the second exit surface refracts the second beam, therefore each of the second beams can be projected to one of the light modulators in the light modulation module. Each light modulator is used to regularly reflect the received second beam to make the second beams returns back to the light guiding element along an original optical path. That is, each light modulator reflects the second beams with a zero-degree angle with the normal. The light guiding element also is used to collimate the second beams reflected by the light modulators to form the illumination light. The illumination light is formed after modulation by the light modulators, which has a certain shape. Processes based on the light guiding element splitting the first beam and combining the second beams is conducive to stitch the fields of view of the second beams reflected by the light modulators, and obtain the illumination light with a larger field of view, wherein an area of the field of view of the illumination light is the sum of areas of the field of views of all the second beams, thus facilitating usage of a field of view of an objective lens in the optical system of the field of view stitching system.

### BRIEF DESCRIPTION OF DRAWINGS

FIG. 1 is a schematic view of a biological sample identification device, a biological sample, a sequencing chip, and a sample platform of an embodiment.
FIG. 2 is a schematic view of an optical path formed by the biological sample identification device, the biological sample, and the sequencing chip in FIG. 1.
FIG. 3 is a perspective view of a three-dimensional structure of a light guiding element in FIG. 2.
FIG. 4 is a front side view of the light guiding element in other embodiments.
FIG. 5 is a top view of a protrusion portion of the light guiding element in other embodiments.
FIG. 6 is a perspective view of another embodiment of the light guiding element in FIG. 2.
FIG. 7 is a planar view of a light modulation module with multiple micro mirrors in FIG. 2.
FIG. 8 shows rotation states of the micro mirrors in FIG. 7.
FIG. 9 is another planar view of the light modulation module in FIG 2.
FIG. 10 shows a light-splitting process of the light guiding element in FIG 2 in a Y dimension.
FIG. 11 shows a light-splitting process of the light guiding element in FIG 2 in an X dimension.
FIG. 12 is a schematic view of a propagation path of second beams from the light guiding element in FIG. 9.
FIG. 13 shows a projection the second beams shown in FIG 12 on a plane from another perspective.
FIG. 14 is a flow chart of a field of view stitching method of an embodiment.
FIG. 15 is a flow chart of a biological sample identification method based on super-resolution imaging of an embodiment.
The following specific embodiments will further illustrate the present disclosure in conjunction with the above drawings.

### DETAILED DESCRIPTION

### Embodiment 1

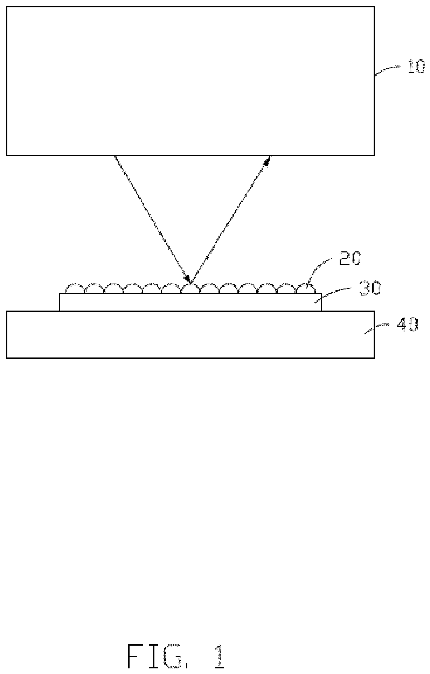

Referring to FIG. 1, a biological sample identification device 10 is used to obtain a biological feature of a biological sample 20. The biological sample 20 may be a nucleic acid sample (DNA or RNA), a protein sample, or a cell sample, etc. In this embodiment, the biological sample 20 is a nucleic acid sample, and the biological feature is a base sequence of the nucleic acid sample.

The biological sample identification device 10 in this embodiment projects laser onto a surface of the biological sample 20 when obtaining the biological feature of the biological sample 20. The biological sample 20 carries a fluorescent labeler, which is stimulated to emit fluorescence when irradiated by the laser. The biological sample identification device 10 is used to receive the fluorescence and generate a detection image based on the fluorescence, and is also used to recognize the detection image to obtain the biological feature of the biological sample 20.

In this embodiment, the biological sample 20 is carried on a sequencing chip 30, and the sequencing chip 30 is carried on a sample platform 40. The biological sample identification device 10 continuously emits the laser during a process of obtaining the biological feature of the biological sample 20, while the sample platform 40 moves horizontally in a plane to drive the sequencing chip 30 and the biological sample 20 to move horizontally in the plane. Therefore, the laser emitted by the biological sample identification device 10 can be projected to different positions on the surface of the biological sample 20. The process of the laser projecting onto different positions on the surface of the biological sample 20 can also be referred as laser scanning of the biological sample 20. In other embodiments, the sample platform 40 may be omitted, while an emission angle of the laser can be changed so that the laser can be projected onto different positions of the biological sample 20.

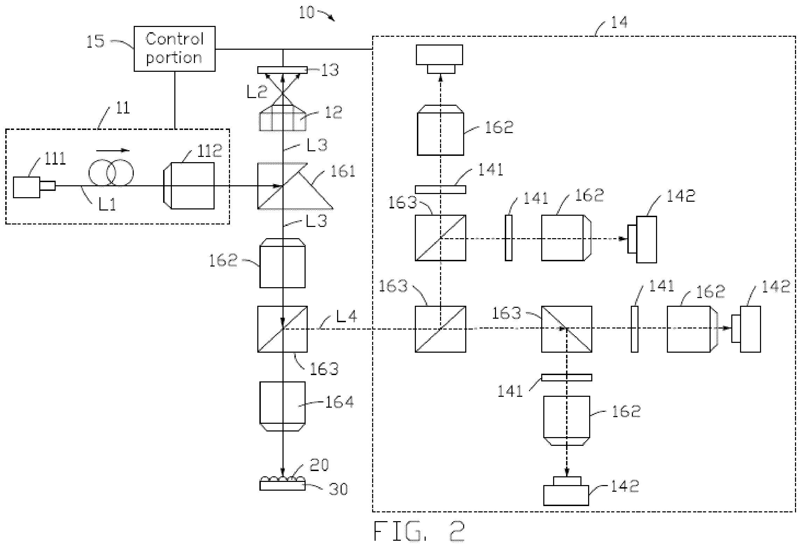

Referring to FIG. 2, the biological sample identification device 10 includes a light source module 11, a light guiding element 12, a light modulation module 13, an imaging module 14, and a control portion 15. The light source module 11, the light modulation module 13, and the imaging module 14 are electrically connected to the control portion 15.

In this embodiment, the light source module 11 includes one laser device 111 and a beam expanding lens 112. The laser device 111 is used to emit a first beam L1, which is laser light. The beam expanding lens 112 is on an optical path of the first beam L1 and is used to expand and collimate the first beam L1 transmitted thereto. In other embodiments, the light source module 11 may include other numbers of laser devices, such as two, four, etc. If a spot area of the first beam L1 emitted by the laser device 111 in the light source module 11 meets requirements of a subsequent optical path, the light source module 11 may also not include the beam expanding lens 112.

The light guiding element 12 is on the optical path of the first beam L1 and is used to split the first beam L1 to generate a plurality of second beams L2.

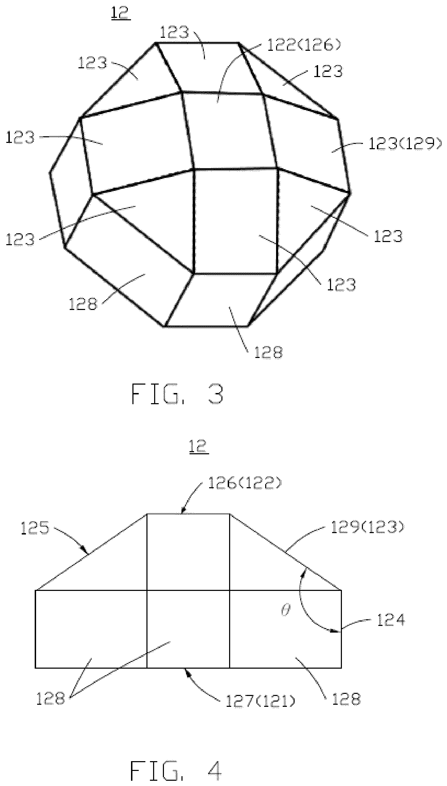

Referring to FIGS. 3 and 4, the light guiding element 12 includes an incident surface 121 positioned corresponding to the light source module 11 for receiving the first beam L1, a first exit surface 122 facing and spaced apart from the incident surface 121, and multiple second exit surfaces 123 bending from edges of the first exit surface 122 and extending towards the incident surface 121. In this embodiment, the first exit surface 122 and the second exit surfaces 123 are interconnected. The second exit surfaces 123 surround the first exit surface 122.

The second beams L2 exit from the first exit surface 122 and the second exit surfaces 123. In this embodiment, one of the second beams L2 is transmitted through the first exit surface 122, and the other second beams L2 are refracted through the second exit surface 123.

In this embodiment, the light guiding element 12 includes one first exit surface 122 and eight second exit surfaces 123, and the light guiding element 12 is used to split the first beam L1 into nine second beam L2. The eight second exit surfaces 123 are interconnected and surround the first exit surface 122. One of the nine second beams L2 directly transmits from the first exit surface 122, while the other eight second beams L2 are refracted by the second exit surfaces 123, respectively.

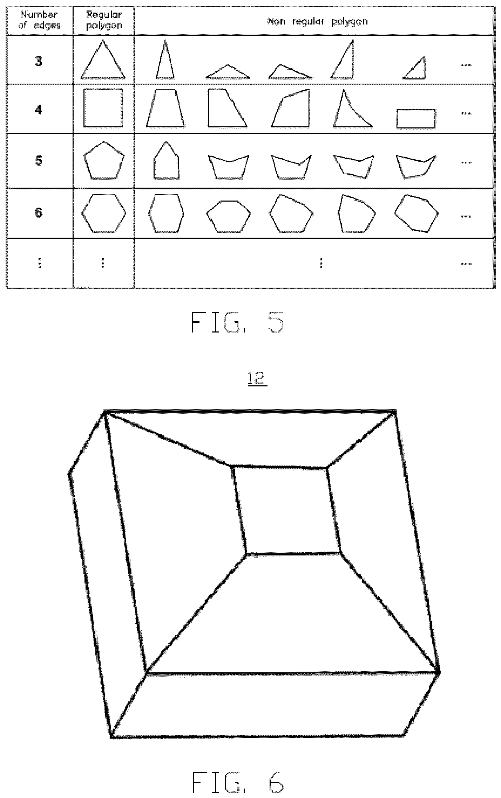

The light guiding element 12 of this embodiment is boss-shaped and includes a stage portion 124 and a protrusion portion 125. The stage portion 124 is in shape of a polygonal prism, and the protrusion portion 125 is connected to an end of the stage portion 124 and coaxial with the stage portion 124. The stage portion 124 includes a bottom surface 127 at an end away from the protrusion portion 125 and a plurality of side surfaces 128 between the bottom surface 127 and the protrusion portion 125. The bottom surface 127 is the incident surface 121 of the light guiding element 12. The protrusion portion 125 includes a plurality of inclined plane surfaces 129 and a top surface 126. The inclined plane surfaces 129 are between the top surface 126 and the stage portion 124, each inclined plane surface 129 intersects and is connected with one side surface 128 (an angle θ between the inclined plane surface129 and the side surface 128 is less than 180°), and each inclined plane surface 129 intersects and connects with the top plane 126. In this embodiment, the top surface 126 is parallel to the bottom surface 127, and an area of the top surface 126 is smaller than an area of the bottom surface 127. The inclined plane surfaces 129 and the top surface 126 cooperatively form the surface of the protrusion portion 125. Each inclined plane surface 129 is one of the second exit surfaces 123, and the top surface 126 is the first exit surface 122. A shape of the top surface 126 (that is, the first exit surface 122) can be the same as or different from the bottom surface 127. For example, the number of edges of the top surface 126 (that is, the first exit surface 122) may be less than the number of edges of the bottom surface 127. In this embodiment, the stage portion 124 is an octagonal prism, the bottom surface 127 (that is, the incident surface 121) is octagonal and has eight sides, the top surface 126 (that is, the first incident surface 122) is quadrilateral and has four sides, the number of the inclined plane surfaces 129 (that is, the second incident surface 123) is the same as the number of edges of the stage portion 124, which is eight, wherein four of the inclined plane surfaces 129 are rectangular (including the case that some of the plane surfaces 129 are square-shaped), the other four of the inclined plane surfaces 129 are triangular, and each second exit surfaces 123 in the shape of a rectangle alternates with one second exit surfaces 123 in the shape of a triangle. Each rectangle-shaped second exit surface 123 is defined as a first refraction surface, and each first refraction surface corresponds to one edge of the first exit surface 122. Each triangle-shaped second exit surface 123 is defined as a second refraction surface, and each second refraction surface corresponds to one corner of the first exit surface 122. In other embodiments, the top surface 126 (that is, the first exit surface 122) can also be in other shapes as shown in FIG. 5. If the number of edges of the first exit surface 122 is represented as M and the number of edges of the stage portion 124 is represented as N, there are two relationships between M and N: (1) N=2M (as shown in FIG. 3); (2) N=M (as shown in FIG. 6).

The nine second beams L2 emitted from the first exit surface 122 and the second exit surfaces 123 of the light guiding element 12 are projected onto a surface of the light modulation module 13 facing the light guiding element 12 (as shown in FIG. 2).

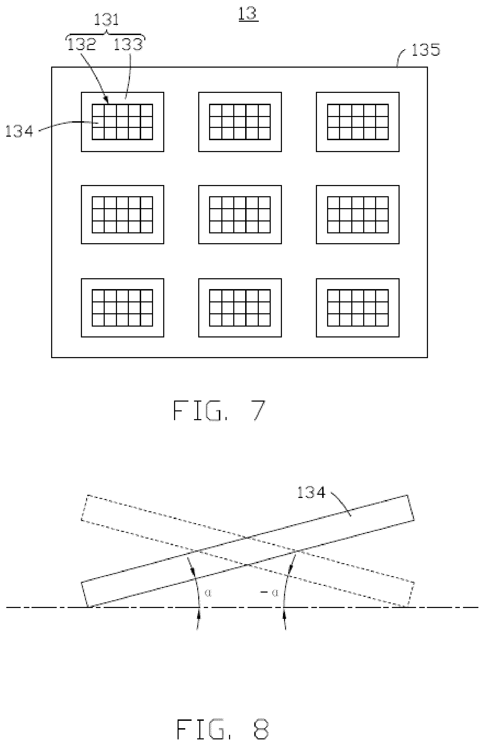

Referring to FIG. 7, the light modulation module 13 includes a plurality of light modulators 131 spaced apart from each other. The number of the light modulators 131 is the same as the number of the second beams L2, and each light modulator 131 is used to receive and regularly reflect one of the second beams L2. That is, each light modulator 131 reflects the second beams L2 with a zero-degree angle with the normal. In this embodiment, nine light modulators 131 are included since there are nine second beams L2. The nine light modulators 131 are arranged in an array with three rows and three columns, spaces between any adjacent two light modulators 131 in each row are equal, and spaces between any adjacent two light modulators 131 in each column are equal. Each light modulator 131 includes an effective light modulation area 132 and a peripheral area 133. The peripheral area 133 surrounds and is connected to the effective light modulation area 132. The effective light modulation area 132 is configured for receiving and reflecting the second beams, and the peripheral area 133 is typically used to carry some necessary structures such as peripheral circuits. The light modulation module 13 also includes a substrate 135, and all the light modulators 131 are carried on a same surface of the substrate 135.

In this embodiment, each light modulator 131 is a digital micromirror device (DMD). Each light modulator 131 includes a plurality of micro mirrors 134 in the effective light modulation area 132. The micro mirrors 134 are arranged in an array including a plurality of rows and columns. In other embodiments, each light modulator 131 may be a spatial light modulator.

Referring to FIG. 8, each micro mirror 134 can be rotated within a certain angle range. In this embodiment, each micro mirror 134 can be rotated about its axis in two opposite directions, and maximum rotation angles in the two opposite directions are the same. The maximum rotation angles in the two opposite directions are defined as α and -α. When the biological sample identification device 10 is in operation, each micro mirror 134 is controlled to be in a state of rotating an angle α or rotating an angle -α. For each micro mirror 134, a state that the rotation angle of the micro mirror 134 is α is defined as a "ON" state, and a state that the rotation angle of the micro mirror 134 is -α is defined as a "OFF" state.

By adjusting the state of each micro mirror 134 in each light modulator 131, the second beams L2 reflected by the light modulators 131 can be modulated into different shapes, so that the nine second beams L2 are combined by the light guiding elements 12 to form illumination light L3 of different shapes and exit through the incident surface 121. In this embodiment, the illumination light L3 is stripe-shaped, which can also be called stripe structured light. A shape of the illumination light L3 is usually determined by a spot shape formed when the illumination light L3 reaching a surface of the biological sample 20. For example, in this embodiment, the illumination light L3 (that is, stripe structured light) has a stripe pattern, which means the light spot formed on the surface of the biological sample 20 when the illumination light L3 reaching the surface of the biological sample 20 is a stripe pattern.

In this embodiment, parameters of the light guiding element 12 and the light modulation modules 13 need to meet the specific conditions, so that each of the nine second beams L2 from the light guiding element 12 covers the effective light modulation area 132 of one of the light modulators 131, and each light modulator 131 can regularly reflect the second beam L2 transmitted thereto.

The specific conditions include that the light spot formed when each second beam L2 reaching the light modulator 131 can completely cover the effective light modulation area 132 of the light modulator 131.

The specific conditions also include that the second beams L2 projected into the effective light modulation area 132 are regularly reflected by the light modulators 131.

Specific structures of the light guiding element 12 and the light modulation modules 13 in this embodiment will be explained as examples.

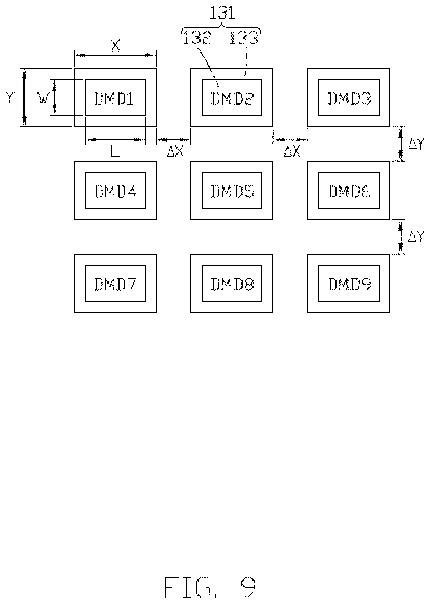

In this embodiment, N=2M=8. Referring to FIG. 9, the light modulation module 13 includes nine light modulators 131 (represented as DMD1, DMD2, DMD3, DMD4, DMD5, DMD6, DMD7, DMD8, and DMD9, respectively in FIG, 9). The effective light modulation area of each light modulator 131 is rectangular with a length L and a width W. A shape of each light modulator 131 (that is, a contour of the peripheral area 133) is in a rectangle shape with a length X and a width Y. In the array of nine light modulators 131, spaces between any adjacent two light modulators 131 in each row are ΔX, and spaces between any adjacent two light modulators 131 in each column are ΔY Referring to FIG. 2, a light outlet of the laser device 111 is rectangular, and the first beam L1 emitted by the laser device 111 can form a rectangular spot with an area of 3L×3W (that is, a sum of the effective light modulation areas 132 of the nine light modulators 131) after being expanded and collimated by the beam expanding lens.

The light guiding element 12 splits the first beam L1 along two dimensions: the X dimension and the Y dimension.

Light Splitting in the Y dimension can be described as follows.

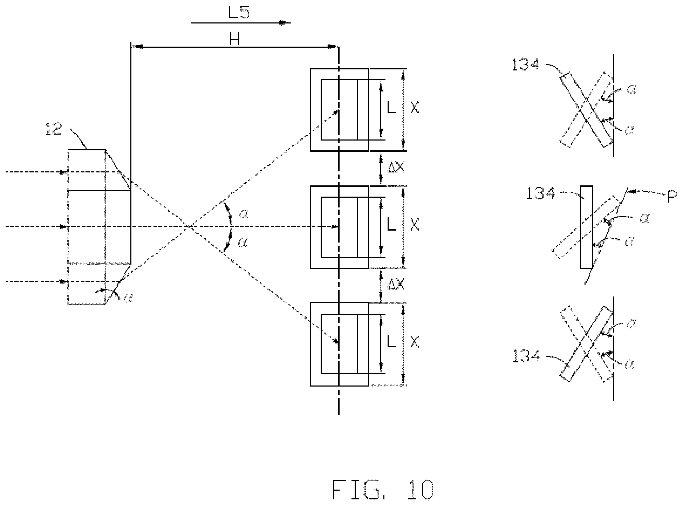

Referring to FIG. 10, in the Y dimension, a size of the first beam L1 is 3L. A direction in which the light guiding element 12 transmits the second beams L2 is defined as a transmission direction. On a plane formed by the transmission direction and the X direction, a cone angle α is formed by the second exit surfaces 122 and the incident surface 121. A refractive index of the light guiding element 12 is n. The transmission direction of the second beams L2 transmitted from the first exit surface 122 is parallel to an optic axis L5. Along a direction of the optic axis L5, a distance between the light guiding element 12 and the light modulation module 13 is H. If following condition (1) is satisfied, the second beams L2 split along the Y direction can be projected onto corresponding DMD.$2 Htan \left[α\left(n-1\right)\right] = 2 X + 2 ΔX + L$ (1)

The angle of the DMD can be further adjusted to ensure that the corresponding DMD can regularly reflected the corresponding second beam L2. That is, a surface of each DMD reflecting the second beam L2 is perpendicular to a reflection direction thereof. The light guiding element 12 is on the plane formed of the transmission direction and the X direction, and a cone angle of the second exit surface 123 and the incident surface 121 is equal to the rotation angle of the DMD. Each DMD for receiving and reflecting the second beam L2 transmitted from the first exit surface 121 has a reference plane P, and the micro mirrors 134 maintain an angle α with the reference plane P in both "ON" state and "OFF" state. In this embodiment, the reference plane P of each DMD needs to be rotated with angle α to ensure the second beams L2 received are regularly reflected, which can also meet the condition (1) above.

Light splitting in the X dimension can be described as follows.

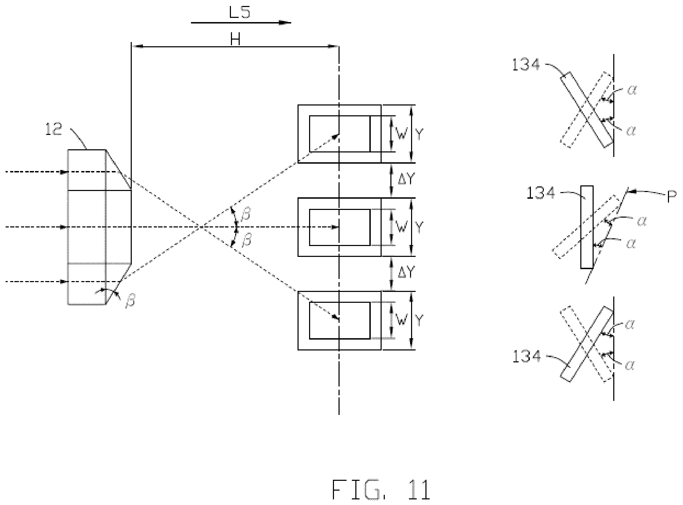

Referring to FIG. 11, in the X dimension, the size of the first beam L1 is 3W, and the light guiding element 12 is on the plane formed by the transmission direction and the Y direction, a cone angle between the second exit surface 123 and the incident surface 121 is β. Under a premise of condition (1), a proper distance H can be obtained. Further adjusting the cone angle β and the spacing ΔY can satisfy the following condition (2), so that the second beams L2 split along the X direction can be projected onto the corresponding DMDs respectively, that is, the condition (2) can be satisfied.$2 Htan \left[β\left(n-1\right)\right] = 2 Y + 2 ΔY + W$ (2)

Further adjusting the angle of the DMD can ensure that the DMD receiving the second beam L2 split along the X direction can reflect the corresponding second beam L2 regularly, as the cone angle is β, the rotation angle of the DMD remains α, it is necessary to rotate the reference plane P of the DMD to ensure regular reflection. The reference planes P of all the DMDs rotate clockwise with an angle of α-β, and the above condition (2) can be satisfied. The reference plane P of the DMD facing the first reflecting surface 121 has rotated in the Y dimension with the angle α, and therefore a total rotation angle is 2α-β, which makes it possible to meet condition (2) in the X dimension as well.

Therefore, based on ΔX and ΔY, an arrangement of the light modulators 131 in the light modulation module 13 can be determined. Furthermore, based on symmetry characteristics and the cone angles α and β of the DMDs in the X direction and the Y direction, a structure of an exit surface (including first exit surface 122, and second exit surface 123) of the light guiding element 12 emitting the second beams L2 can be obtained.

In other embodiments of the present disclosure, for the light modulation module 13 formed by light modulators 131 in other irregularly arrangement, the output surface of the light guiding element 12 can be designed in a shape different from the shape described in the present embodiment according to actual splitting needs, as long as positions of the DMDs can be adjusted to ensure that each second beam L2 can cover the effective light modulation area 132 of a matched DMD and achieve regular reflection by the DMD.

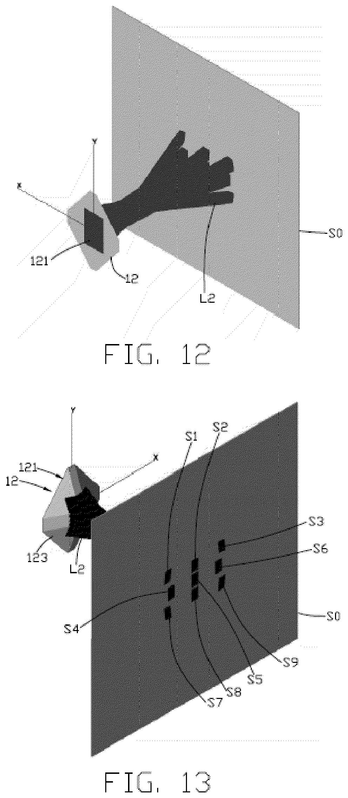

FIG. 12 shows a propagation path of the second beams L2 from the light guiding element 12 shown in FIG. 9, which satisfies the conditions (1) - (2) above. FIG. 13 shows a projection pattern of the second beams L2 shown in FIG. 12 on a projection plane S0 from another perspective. According to results shown in FIG.S 12 to 13, the propagation path of the second beam L2 is basically the same as that shown in FIG.S 10 and 11. The projection plane S0 is used to represent the plane used by the light modulation module 13 to receive the second beams L2. According to FIG. 13, the nine second beams L2 form projection patterns S1, S2, S3, S4, S5, S6, S7, S8, and S9 on the projection plane S0. An arrangement of the projection patterns S1, S2, S3, S4, S5, S6, S7, S8, and S9 is the same with the arrangement of the light modulators 131 in the light modulation module 13 (referring to FIG. 9), which enables each second beam L2 to be correspondingly projected onto the light modulator 131 by the light guiding element 12.

Referring to FIG. 2, after being regularly reflected by the light modulation module 13, the second beams L2 are once again received and combined by the light guiding element 12 to form the illumination light L3. The illumination light L3 can be guided to the biological sample 20 through the incident surface 121.

The illumination light L3 irradiates the surface of the biological sample 20 to excite biological sample 20 to emit fluorescence light, and the fluorescence light emitted by biological sample 20 is defined as a detection light L4. In this embodiment, the imaging module 14 is used to receive the detection light L4 and obtain a detection image of the biological sample 20 based on the detection light L4.

In this embodiment, the biological sample 20 is excited by the illumination light L3 to generate four different wavelengths of fluorescence lights, that is, the detection light L4 includes four different wavelengths of fluorescence lights. The imaging module 14 includes four imaging channels, each of which is used to receive a fluorescence light of one of the wavelengths (i.e., each imaging channel is used to receive a part of detection light L4). In other embodiments, the imaging module 14 may include other numbers of imaging channels, which are determined by the number of fluorescence wavelengths in the detection light L4. Specifically, each imaging channel corresponds to one wavelength of the fluorescence light. That is, fluorescence light of each wavelength correspondingly gets transmitted onto one single imaging channel.

Each imaging channel includes a filter 141 and a camera 142 arranged sequentially on an optical path of the detection light L4. The filter 141 allows only the fluorescent light of one wavelength (or wavelength band) to pass through and get transmitted onto the camera 142. The camera 142 can generate the detection image based on the fluorescence light received, which can be used to obtain the biological feature of the biological sample 20.

The control portion 15 is electrically connected to the laser device 111 in the light source module 11, each light modulator 131 in the light modulation module 13, and each camera in the imaging module 14, which is configured for controlling switch state of the laser device 111 in the light source module 11, the rotation state ("ON" state or "OFF" state) of the micro mirrors 134 in the light modulator 131, and an operation process of each camera. The control portion 15 is also used to identify the biological feature of the biological sample based on preset algorithms according to the detection image obtained as mentioned above. The control portion 15 can be a computer, a control chipset, or the like.

The biological sample identification device 10 of this embodiment also includes a light guiding device for guiding light (including the first beam L1, the second beams L2, the illumination light L3, and the detection light L4) in a specific way. The light guiding device includes a total internal reflector 161, five lenses 162, four dichroic mirrors 163, and an objective lens 164. The total internal reflector 161 is positioned in both an optical path the first beam L1 and that of the illumination light L3, and configured to guide the first beam to the light guiding element 12, and to guide the illumination light L3 to the biological sample 20 through lens 162, dichroic mirror 163, and the objective lens 164, by expanding, evenly distributing, and transmitting a received light. Each of the other four lenses 162 are positioned between the filter 141 and the camera 142 in a imaging channel, for focusing the fluorescence light onto the camera 142. Each dichroic mirror 163 is used to reflect or transmit received light according to the wavelength of the received light. The objective lens 165 is used to focus the illumination light L3 onto the biological sample 20 (or onto the sequencing chip 30).

The above light guiding devices are only used as examples and are not intended to limit the present disclosure. In other implementations, the above light guiding devices may be replaced by other types of light guiding devices or a combination of a plurality of other light guiding devices that can achieve a same guiding effect on light.

In the biological sample identification device 10 of this embodiment, the light guiding element 12 includes a first exit surface 122 and a plurality of second exit surfaces 123. The light guiding element 12 is used to split the first beam L1 emitted by the light source module 11, so that the first exit surface 122 and each second exit surface 123 respectively emit one of the second beams L2 to project each second beam L2 onto one light modulator 131 in the light modulation module 13. The light modulator 131 is used for regularly reflecting the second beam L2 received, causing each second beam L2 to return to the light guiding element 12 along an original optical path. The light guiding element 12 is used for combining the second beams L2 reflected by the light modulators 131 to form the illumination light L3, and the field of view area of the illumination light L3 is a sum of the field of view areas of the second beams L2. In another embodiment of this disclosure, the field of view area of the illumination light L3 may not be the sum of the field of view areas of the second beams L2, and the field of view area of the illumination light L3 is greater than or equal to the field of view area of any one of second beams L2. When the field of view area of the illumination light L3 is the sum of the field of view areas of the second beams L2, the field of view areas of the second beams L2 can be greatly utilized.

Compared to prior art, the biological sample identification device 10 in this disclosure splits the first beam L1 and combines the second beams L2 by the light guiding element 12, further cooperates with the light modulators 131 to obtain the illumination light L3 with a larger field of view area. In the biological sample identification device 10, the field of view area of the objective lens 164 is usually larger than that of light reflected by one light modulator 131. Therefore, by increasing the number of the light modulators 131, the illumination light L3 with a field of view area several times larger than a field of view area of one second beam L2 is obtained, which is conducive to match the field of view area of the objective lens 164 and making full use of it. The field of view area of the illumination light L3 can be slightly larger, or slightly smaller, or equal to the field of view area of the objective lens 164. When the field of view area of the illumination light L3 is slightly greater than or equal to the field of view area of the objective lens 164, the maximum utilization of the field of view area of the objective lens 164 can be achieved. When the field of view area of the illumination light L3 is slightly smaller than that of the objective lens 164, it is beneficial to avoid waste of illumination light L3 and thus improve a utilization rate of the illumination light L3.

The field of view area of light beam (such as the second beams L2 and the illumination light L3) described in this embodiment can be defined as the spot area formed when the light beam reaches the biological sample 20. The field of view area of the objective 165 is determined by a field angle of the objective 165.

The light source module 11, the light guiding element 12, and the light modulation module 13 can form a field of view stitching system, which can be applied to other optical systems except for super-resolution imaging device 10, such as laser projection or computational lithography. Based on a process of splitting the first beam L1 and combining the second beams L2 by the light guiding element 12 in the field of view stitching system, the light modulation module 13 can include a plurality of light modulators 131, which is conducive to splicing the field of view of the second beams L2 reflected by the light modulators 131, and obtaining the illumination light with a larger field of view to fully utilize the field of view of the objective lens in the optical system.

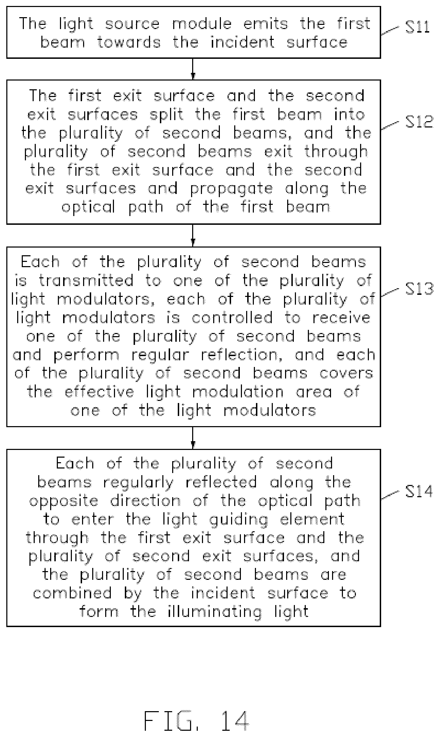

Referring to FIG. 14, this embodiment also provides a field of view stitching method, which is applied to the field of view stitching system. The field of view stitching method includes the following steps.

In step S11, the light source module emits the first beam towards the incident surface.

In step S12, the first exit surface and the second exit surfaces split the first beam into the plurality of second beams, and the plurality of second beams exit through the first exit surface and the second exit surfaces and propagate along the optical path of the first beam.

In step S13, each of the plurality of second beams is transmitted to one of the plurality of light modulators, each of the plurality of light modulators is controlled to receive one of the plurality of second beams and perform regular reflection, and each of the plurality of second beams covers the effective light modulation area of one of the light modulators.

In step S14, each of the plurality of second beams regularly reflected along the opposite direction of the optical path to enter the light guiding element through the first exit surface and the plurality of second exit surfaces, and the plurality of second beams are combined by the incident surface to form the illuminating light.

The steps S11, S12, S13, and S14 in the above field of view stitching method correspond to operation processes of the light source module 11, the light guiding element 12, and the light modulation module 13 in the field of view stitching system and the operation processes will not be repeated here.

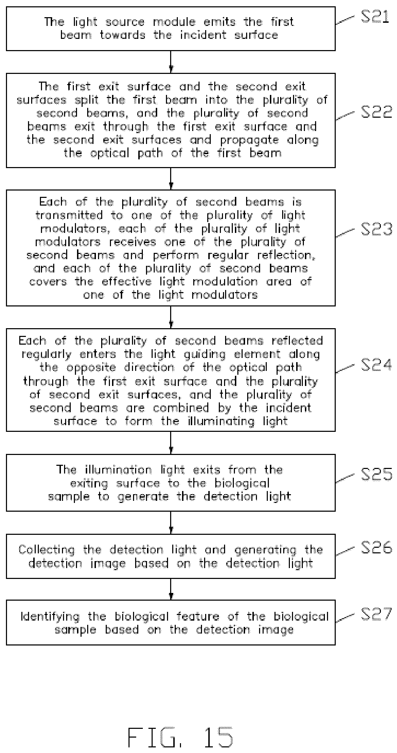

Referring to FIG. 15, this embodiment also provides a biological sample identification method based on super-resolution imaging, which is applied to the biological sample identification device 100 based on super-resolution imaging. The biological sample identification method based on super-resolution imaging applied with the steps in the field of view stitching method. The biological sample identification method based on super-resolution imaging specifically includes the following steps.

In step S21, the light source module emits the first beam towards the incident surface.

In step S22, the first exit surface and the second exit surfaces split the first beam into the plurality of second beams, and the plurality of second beams exit through the first exit surface and the second exit surfaces and propagate along the optical path of the first beam.

In step S23, each of the plurality of second beams is transmitted to one of the plurality of light modulators, each of the plurality of light modulators receives one of the plurality of second beams and perform regular reflection, and each of the plurality of second beams covers the effective light modulation area of one of the light modulators.

In step S24, each of the plurality of second beams reflected regularly enters the light guiding element along the opposite direction of the optical path through the first exit surface and the plurality of second exit surfaces, and the plurality of second beams are combined by the incident surface to form the illuminating light.

In step S25, the illumination light exits from the exiting surface to the biological sample to generate the detection light.

In step S26, the method includes collecting the detection light and generating the detection image based on the detection light.

In step S27, the method includes identifying the biological feature of the biological sample based on the detection image.

The steps S21, S22, S23, S24, S25, S26, and S27 in the biological sample identification method based on super-resolution imaging correspond to operation processes of the light source module 11, the light guide element 12, the light modulation module 13, the imaging module 14, and the control portion 15 in the biological sample identification device 100 based on super-resolution imaging, and the operation processes will not be repeated here.

The above field of view stitching method and the biological sample identification method based on super-resolution imaging can achieve all the beneficial effects of the field of view stitching system and the biological sample identification device based on super-resolution imaging.

Ordinary technicians in the technical field should realize that the above embodiments are only used to illustrate the present disclosure and not to limit the present disclosure. Appropriate changes made to the Z above embodiments fall within a protection scope of the present disclosure as long as the changes are within the scope of the claims.

## Claims

1. A field of view stitching system comprising:
a light source module (11) for emitting a first beam(L1);
a light guiding element (12) on an optical path of the first beam (L1) and configured to split the first beam(L1) into a plurality of second beams(L2), wherein the light guiding element(12) comprises an incident surface (121) positioned corresponding to the light source module(11), a first exit surface(122) facing and spaced apart from the incident surface(121), and a plurality of second exit surfaces (123) bending and extending from edges of the first exit surface(122) towards the incident surface(121), the plurality of second beams(L2) exit from the first exit surface (122) and the plurality of second exit surfaces (123) and propagate along the optical path; and
a light modulation module(13) comprising a plurality of light modulators(131), wherein, a number of the plurality of light modulators (131) is the same as a total number of the first exit surface (122) and the plurality of second exit surfaces(123), each of the plurality of light modulators (131) is configured to receive and regularly reflect one of the plurality of second beams(L2), each of the plurality of light modulators (131) has an effective light modulation area(132), each of the plurality of second beams (L2) covers the effective light modulation area (132) of one of the light modulators (131) when incident on the light modulator(131);
wherein the light guiding element (12) is further configured for receiving and combining the plurality of second beams (L2) reflected by the plurality of light modulators (131) to form an illuminating light (L3) to further exit through the incident surface (121).

2. The field of view stitching system of claim 1, wherein one of the plurality of second beams (L2) exit from the first exit surface(122), and other second beams (L2) of the plurality of second beams (L2) exit from the plurality of second exit surfaces (123), the first exit surface(122) is connected to the plurality of second exit surfaces(123), and the plurality of second exit surfaces (123) surround the first exit surface(122).

3. The field of view stitching system of claim 2, wherein the light guiding element (12) comprises a stage portion(124) and a protrusion portion(125), the protrusion portion(125) is connected to an end of the stage portion(124) and coaxial with the stage portion(124), a surface of the protrusion portion(125) comprises the first exit surface (122) and the plurality of second exit surfaces(123), a surface of the stage portion (124) away from the protrusion portion(125) is the incident surface(121), and the incident surface(121) is parallel to the first exit surface(122).

4. The field of view stitching system of claim 3, wherein the first exit surface(122) is polygonal;
a number of the plurality of second exit surfaces (123) equals a number of sides of the first exit surface(122); or a number of the plurality of second exit surfaces (123) is twice a number of sides of the first exit surface(122), the second exit surface(123) comprises a plurality of first refraction surfaces and a plurality of second refraction surfaces, with a one-to-one correspondence between the plurality of first refraction surfaces and sides of the first exit surface(122), and a one-to-one correspondence between the plurality of second refraction surfaces and corners of the_first exit surface(122), and each of the plurality of second refraction surfaces is formed between any two adjacent first refraction surfaces.

5. The field of view stitching system of any one of claims 3-4, wherein the stage portion (124) is in shape of a cylinder with a plurality of arris, and a number of the plurality of arris is the same as a number of the plurality of second exit surfaces(123).

6. The field of view stitching system of any one of claims 1-4, wherein each of the plurality of light modulators(131) comprises a plurality of micro mirrors in the effective light modulation area(132), each of the plurality of micro mirrors has a rotation angle α, the effective light modulation area(132) of each of the plurality of light modulators(131) is rectangular with a length L and a width W, a length of each of the plurality of light modulators (131) is X, a width of each of the plurality of light modulators(131) is Y, the plurality of second exit surfaces(123) surround the first exit surface(122) and are axially symmetric around an axis in an X direction and another axis in a Y direction, the X direction is parallel to a length direction of the plurality of light modulators(131), and the Y direction is parallel to a width direction of the plurality of light modulators(131), the plurality of light modulators(131) are configured in an array arrangement with a plurality of rows with a same number of the light modulators(131), having a space ΔX between every adjacent two of the light modulators along the X direction; on a plane formed by the X direction and an optic axis of the light guiding element(12), a cone angle between the incident surface(121) and the plurality of second exit surfaces(123) corresponding to the plurality of light modulators(131) arranged along the X direction is α; a refractive index of the light guiding element(12) is n, a distance between the first exit surface(122) and the light modulation module (13) along the optic axis is H, and following condition is satisfied: $2 Htan \left[α\left(n-1\right)\right] = 2 X + 2 ΔX + L .$

7. The field of view stitching system of claim 6, wherein a distance between adjacent light modulators(131) along the Y direction is ΔY;
on a plane formed by the optic axis and the Y direction, a cone angle between the plurality of second exit surfaces(123) and the incident surface(121) corresponding to the plurality of light modulators(131) along the Y direction is β, and the following condition is satisfied: $2 Htan \left[β\left(n-1\right)\right] = 2 Y + 2 ΔY + W .$

8. The field of view stitching system of any one of claims 1-4, wherein the light source module(11) comprises at least one laser device(111) and at least one beam expanding lens(112), the at least one beam expanding lens(112) is positioned between the at least one laser device(111) and the light guiding element(12);
the at least one laser device(111) is configured to emit the first beam(L1), the at least one beam expanding lens(112) is configured to receive, expand, and collimate the first beam(L1), and a spot area of the first beam(L1) after being expanded and collimated by the at least one beam expanding lens(112) is a sum of the effective light modulation area(132) of the plurality of light modulators(131).

9. The field of view stitching system of any one of claims 1-4, wherein a field of view area of the illumination light(L3) is greater than a field of view area of any one of the plurality of second beams(L2).

10. A biological sample identification device(10) based on super-resolution imaging, comprising:
a field of view stitching system according to any of claims 1-9;
an imaging module(14) for receiving the detection light and generating a detection image based on the detection light; and
a control portion electrically connected to the light source module(11), the plurality of light modulators(131), and the imaging module(14), the control portion is configured to control an operation process of the light source module(11), the plurality of light modulators(131), and the imaging module(14), and to identify a biological feature of the biological sample based on the detection image.

11. The biological sample identification device based on super-resolution imaging of claim 10, wherein each of the plurality of light modulators(131) comprises a plurality of micro mirrors in the effective light modulation area(132), each of the plurality of micro mirrors has a rotation angle α, the effective light modulation area(132) of each of the plurality of light modulators(131) is rectangular with a length L and a width W, a length of each of the plurality of light modulators(131) is X, a width of each of the plurality of light modulators(131) is Y, the plurality of second exit surfaces(123) surround the first exit surface(122) and are axially symmetric around an axis in an X direction and another axis in a Y direction, the X direction is parallel to a length direction of the plurality of light modulators(131), and the Y direction is parallel to a width direction of the plurality of light modulators(131), the plurality of light modulators are configured in an array arrangement with a plurality of rows with a same number of the light modulators(131), having a space ΔX between every adjacent two of the light modulators along the X direction; on a plane formed by the X direction and an optic axis of the light guiding element(12), a cone angle between the incident surface(121) and the plurality of second exit surfaces(123) corresponding to the plurality of light modulators arranged along the X direction is α; a refractive index of the light guiding element(12) is n, a distance between the first exit surface(122) and the light modulation module(13) along the optic axis is H, and the following condition is satisfied: $2 Htan \left[α\left(n-1\right)\right] = 2 X + 2 ΔX + L .$

12. The biological sample identification device based on super-resolution imaging of claim 11, wherein a distance between adjacent light modulators(131) along the Y direction is ΔY;
on a plane formed by the optic axis and the Y direction, a cone angle between the plurality of second exit surfaces(123) and the incident surface(121) corresponding to the plurality of light modulators(131) along the Y direction is β, and the following condition is satisfied: $2 Htan \left[β\left(n-1\right)\right] = 2 Y + 2 ΔY + W .$

13. A field of view stitching method applied to a field of view stitching system, wherein the field of view stitching system includes a light source module(11), a light guiding element(12), and a light modulation module(13), the light modulation module(13) is provided with a plurality of light modulators(131), the light guiding element(12) is provided with an incident surface(121), a first exit surface(122) facing the incident surface(121), and a plurality of second exit surfaces(123) bending and extending from edges of the first exit surface(122) towards the incident surface(121), a number of the plurality of light modulators(131) is the same as a total number of the first exit surface(122) and the plurality of second exit surfaces(123), and each of the plurality of light modulators(131) has an effective light modulation area(132); the field of view stitching method comprises:
emitting a first beam(L1) towards the incident surface(121) by the light source module(11);
splitting the first beam(L1) into a plurality of second beams(L2) by the first exit surface(122) and the plurality of second exit surfaces(123), and further directing the plurality of second beams(L2) to exit from the first exit surface(122) and the plurality of second exit surfaces(123) and propagate along an optical path of the first beam(L1);
transmitting each of the plurality of second beams(L2) to one of the plurality of light modulators(131), controlling each of the plurality of light modulators(131) to receive and regularly reflect one of the plurality of second beams(L2), and with each of the plurality of second beams(L2) covering the effective light modulation area(132) of the light modulator(131); and
forming an illumination light(L3) on the incident surface(121) by a combination of the plurality of second beams(L2) which are regularly reflected to the light guiding element(12) along an opposite direction of the optical path and pass through the first exit surface(122) and the plurality of second exit surfaces(123).

14. The field of view stitching method of claim 13, wherein each of the plurality of light modulators(131) is provided with a plurality of micro mirrors in the effective light modulation area(132), a rotatable angle of each of the plurality of micro mirrors is α, the effective light modulation area(132) is rectangular with a length of L and a width of W, each of the plurality of light modulators(131) has a length of X and a width of Y, the plurality of second exit surfaces(123) are symmetrically distributed about the first exit surface(122) and around an axis in an X direction and another axis in a Y direction, the X direction is parallel to a length direction of the plurality of light modulators(131), the Y direction is parallel to a width direction of the plurality of light modulators(131), the plurality of light modulators(131) are arranged in a symmetrical array along the X direction and the Y direction; on a plane formed by an optic axis of the light guiding element(12) and the X direction, the field of view stitching method further includes:
adjusting space between each adjacent two of the light modulators(131) to generate the illumination light(L3), including: adjusting the space between each adjacent two of the light modulators(131) along the X direction, and adjusting a distance H between the first exit surface(122) and the light modulation module(13) along the optic axis; the space between each adjacent two light modulators(131) along the X direction is ΔX, a cone angle between the incident surface and the plurality of second exit surfaces corresponding to the plurality of light modulators arranged along the X direction is α, the cone angle α equals the rotation angle α, and a condition of 2Htan[α(n-1)]=2X+2ΔX+L is satisfied;
and/or
adjusting the space between each adjacent two of the light modulators(131) along the Y direction according to a condition of 2Htan[β(n-1)]=2Y+2ΔY+W, wherein the space between each adjacent two of the light modulators(131) along the Y direction is ΔY; and on a plane formed by an optic axis and the Y direction, a cone angle between the incident surface(121) and the plurality of second exit surfaces(123) corresponding to the plurality of light modulators(131) arranged along the Y direction is β.

15. A biological sample identification method based on super-resolution imaging and is applied to a biological sample identification device, the biological sample identification device is provided with a field of view stitching system including a light source module(11), a light guiding element(12), and a light modulation module(13), the light modulation module(13) is provided with a plurality of light modulators(131), the light guiding element(12) is provided with an incident surface(121) positioned corresponding to the light source module(11), a first exit surface(122) facing and positioned apart from the incident surface(121), and a plurality of second exit surfaces(123) bending and extending from edges of the first exit surface(122) towards the incident surface(121), a number of the plurality of light modulators(131) is the same as a total number of the first exit surface(122) and the plurality of second exit surfaces(123), and each of the plurality of light modulators(131) has an effective light modulation area(132); the biological sample identification method based on super-resolution imaging comprises:
a field of view stitching method according to claim 13 or 14;
irradiating a biological sample with the illumination light(L3) through the incident surface(121) to generate a detection light from the biological sample;
collecting the detection light and generating a detection image based on the detection light; and
identifying a biological feature of the biological sample based on the detection image.

16. The biological sample identification method based on super-resolution imaging of claim 15, wherein each of the plurality of light modulators(131) is provided with a plurality of micro mirrors in the effective light modulation area(132), a rotatable angle of each of the plurality of micro mirrors is α, the effective light modulation area(132) is rectangular with a length of L and a width of W, each of the plurality of light modulators(131) has a length of X and a width of Y, the plurality of second exit surfaces(123) are symmetrically distributed about the first exit surface(122) and around an axis in an X direction and another axis in a Y direction, the X direction is parallel to a length direction of the plurality of light modulators(131), the Y direction is parallel to a width direction of the plurality of light modulators(131), the plurality of light modulators(131) are arranged in a symmetrical array along the X direction and the Y direction; on a plane formed by an optic axis of the light guiding element(12) and the X direction, the field of view stitching method further includes:
adjusting space between each adjacent two of the light modulators(131) to generate the illumination light(L3), including: adjusting the space between each adjacent two of the light modulators(131) along the X direction, and adjusting a distance H between the first exit surface(122) and the light modulation module(13) along the optic axis; wherein the space between each adjacent light modulators(131) along the X direction is ΔX, a cone angle between the incident surface(121) and the plurality of second exit surfaces(123) corresponding to the plurality of light modulators(131) arranged along the X direction is α, the cone angle α equals the rotation angle α, and a condition of 2Htan[α(n-1)]=2X+2ΔX+L is satisfied;
and/or
adjusting the space between each adjacent two of the light modulators(131) along the Y direction according to a condition 2Htan[β(n-1)]=2Y+2ΔY+W, wherein the space between each adjacent two of the light modulators(131) along the Y direction is ΔY; and on a plane formed by an optic axis and the Y direction, a cone angle between the incident surface(121) and the plurality of second exit surfaces(123) corresponding to the plurality of light modulators (131) arranged along the Y direction is β.

## Patentansprüche

1. Ein Sichtfeld-Stitching-System, umfassend:
ein Lichtquellenmodul (11) zum Emittieren eines ersten Strahls (L1);
ein Lichtführungselement (12) auf einem optischen Pfad des ersten Strahls (L1) und konfiguriert, um den ersten Strahl (L1) in eine Vielzahl von zweiten Strahlen (L2) aufzuteilen, wobei das Lichtführungselement (12) eine Eintrittsfläche (121), die korrespondierend zu dem Lichtquellenmodul (11) positioniert ist, eine erste Austrittsfläche (122), die der Eintrittsfläche (121) zugewandt und von dieser beabstandet ist, und eine Vielzahl von zweiten Austrittsflächen (123) umfasst, die sich biegen und von Rändern der ersten Austrittsfläche (122) in Richtung der Eintrittsfläche (121) erstrecken, wobei die Vielzahl von zweiten Strahlen (L2) aus der ersten Austrittsfläche (122) und der Vielzahl von zweiten Austrittsflächen (123) austritt und sich entlang des optischen Pfads ausbreitet; und
ein Lichtmodulationsmodul (13), das eine Vielzahl von Lichtmodulatoren (131) umfasst, wobei eine Anzahl der Vielzahl von Lichtmodulatoren (131) gleich einer Gesamtzahl der ersten Austrittsfläche (122) und der Vielzahl von zweiten Austrittsflächen (123) ist, wobei jeder der Vielzahl von Lichtmodulatoren (131) konfiguriert ist, um einen der Vielzahl von zweiten Strahlen (L2) zu empfangen und regulär zu reflektieren, wobei jeder der Vielzahl von Lichtmodulatoren (131) eine effektive Lichtmodulationsfläche (132) aufweist, wobei jeder der Vielzahl von zweiten Strahlen (L2) die effektive Lichtmodulationsfläche (132) von einem der Lichtmodulatoren (131) abdeckt, wenn er auf den Lichtmodulator (131) einfällt;
wobei das Lichtführungselement (12) ferner zum Empfangen und Kombinieren der Vielzahl von zweiten Strahlen (L2), die von der Vielzahl von Lichtmodulatoren (131) reflektiert werden, konfiguriert ist, um ein Beleuchtungslicht (L3) zu bilden, das weiter durch die Eintrittsfläche (121) austritt.

2. Das Sichtfeld-Stitching-System nach Anspruch 1, wobei einer der Vielzahl von zweiten Strahlen (L2) aus der ersten Austrittsfläche (122) austritt und andere zweite Strahlen (L2) der Vielzahl von zweiten Strahlen (L2) aus der Vielzahl von zweiten Austrittsflächen (123) austreten, wobei die erste Austrittsfläche (122) mit der Vielzahl von zweiten Austrittsflächen (123) verbunden ist und die Vielzahl von zweiten Austrittsflächen (123) die erste Austrittsfläche (122) umgibt.

3. Das Sichtfeld-Stitching-System nach Anspruch 2, wobei das Lichtführungselement (12) einen Stufenabschnitt (124) und einen Vorsprungsabschnitt (125) umfasst, wobei der Vorsprungsabschnitt (125) mit einem Ende des Stufenabschnitts (124) verbunden und koaxial zu dem Stufenabschnitt (124) ist, wobei eine Oberfläche des Vorsprungsabschnitts (125) die erste Austrittsfläche (122) und die Vielzahl von zweiten Austrittsflächen (123) umfasst, wobei eine von dem Vorsprungsabschnitt (125) abgewandte Oberfläche des Stufenabschnitts (124) die Eintrittsfläche (121) ist und die Eintrittsfläche (121) parallel zu der ersten Austrittsfläche (122) ist.

4. Das Sichtfeld-Stitching-System nach Anspruch 3, wobei die erste Austrittsfläche (122) polygonal ist;
eine Anzahl der Vielzahl von zweiten Austrittsflächen (123) gleich einer Anzahl von Seiten der ersten Austrittsfläche (122) ist; oder eine Anzahl der Vielzahl von zweiten Austrittsflächen (123) das Zweifache einer Anzahl von Seiten der ersten Austrittsfläche (122) beträgt, wobei die zweite Austrittsfläche (123) eine Vielzahl von ersten Brechungsflächen und eine Vielzahl von zweiten Brechungsflächen umfasst, mit einer Eins-zu-Eins-Entsprechung zwischen der Vielzahl von ersten Brechungsflächen und Seiten der ersten Austrittsfläche (122) und einer Eins-zu-Eins-Entsprechung zwischen der Vielzahl von zweiten Brechungsflächen und Ecken der ersten Austrittsfläche (122), und jede der Vielzahl von zweiten Brechungsflächen zwischen zwei beliebigen benachbarten ersten Brechungsflächen ausgebildet ist.

5. Das Sichtfeld-Stitching-System nach einem der Ansprüche 3 bis 4, wobei der Stufenabschnitt (124) die Form eines Zylinders mit einer Vielzahl von Kanten aufweist und eine Anzahl der Vielzahl von Kanten gleich einer Anzahl der Vielzahl von zweiten Austrittsflächen (123) ist.

6. Das Sichtfeld-Stitching-System nach einem der Ansprüche 1 bis 4, wobei jeder der Vielzahl von Lichtmodulatoren (131) eine Vielzahl von Mikrospiegeln in der effektiven Lichtmodulationsfläche (132) umfasst, jeder der Vielzahl von Mikrospiegeln einen Rotationswinkel α aufweist, die effektive Lichtmodulationsfläche (132) jedes der Vielzahl von Lichtmodulatoren (131) rechteckig mit einer Länge L und einer Breite W ist, eine Länge jedes der Vielzahl von Lichtmodulatoren (131) X ist, eine Breite jedes der Vielzahl von Lichtmodulatoren (131) Y ist, die Vielzahl von zweiten Austrittsflächen (123) die erste Austrittsfläche (122) umgibt und axialsymmetrisch um eine Achse in einer X-Richtung und eine weitere Achse in einer Y-Richtung ist, die X-Richtung parallel zu einer Längsrichtung der Vielzahl von Lichtmodulatoren (131) ist, und die Y-Richtung parallel zu einer Breitenrichtung der Vielzahl von Lichtmodulatoren (131) ist, die Vielzahl von Lichtmodulatoren (131) in einer Array-Anordnung mit einer Vielzahl von Reihen mit einer gleichen Anzahl von Lichtmodulatoren (131) konfiguriert ist, die einen Abstand ΔX zwischen jeweils zwei benachbarten Lichtmodulatoren entlang der X-Richtung aufweist; auf einer Ebene, die durch die X-Richtung und eine optische Achse des Lichtführungselements (12) gebildet wird, ein Kegelwinkel zwischen der Eintrittsfläche (121) und der Vielzahl von zweiten Austrittsflächen (123) korrespondierend zu der Vielzahl von Lichtmodulatoren (131), die entlang der X-Richtung angeordnet sind, α ist; ein Brechungsindex des Lichtführungselements (12) n ist, ein Abstand zwischen der ersten Austrittsfläche (122) und dem Lichtmodulationsmodul (13) entlang der optischen Achse H ist, und die folgende Bedingung erfüllt ist: $2 Htan \left[α\left(n-1\right)\right] = 2 X + 2 ΔX + L .$

7. Das Sichtfeld-Stitching-System nach Anspruch 6, wobei ein Abstand zwischen benachbarten Lichtmodulatoren (131) entlang der Y-Richtung ΔY ist;
auf einer Ebene, die durch die optische Achse und die Y-Richtung gebildet wird, ein Kegelwinkel zwischen der Vielzahl von zweiten Austrittsflächen (123) und der Eintrittsfläche (121) korrespondierend zu der Vielzahl von Lichtmodulatoren (131) entlang der Y-Richtung β ist, und die folgende Bedingung erfüllt ist: $2 Htan \left[β\left(n-1\right)\right] = 2 Y + 2 ΔY + W .$

8. Das Sichtfeld-Stitching-System nach einem der Ansprüche 1 bis 4, wobei das Lichtquellenmodul (11) mindestens eine Laservorrichtung (111) und mindestens eine Strahlaufweitungslinse (112) umfasst, wobei die mindestens eine Strahlaufweitungslinse (112) zwischen der mindestens einen Laservorrichtung (111) und dem Lichtführungselement (12) positioniert ist;
die mindestens eine Laservorrichtung (111) konfiguriert ist, um den ersten Strahl (L1) zu emittieren, die mindestens eine Strahlaufweitungslinse (112) konfiguriert ist, um den ersten Strahl (L1) zu empfangen, aufzuweiten und zu kollimieren, und eine Spotfläche des ersten Strahls (L1) nach dem Aufweiten und Kollimieren durch die mindestens eine Strahlaufweitungslinse (112) eine Summe der effektiven Lichtmodulationsfläche (132) der Vielzahl von Lichtmodulatoren (131) ist.

9. Das Sichtfeld-Stitching-System nach einem der Ansprüche 1 bis 4, wobei eine Sichtfeldfläche des Beleuchtungslichts (L3) größer als eine Sichtfeldfläche eines beliebigen der Vielzahl von zweiten Strahlen (L2) ist.

10. Eine Vorrichtung zur Identifizierung biologischer Proben (10) basierend auf superauflösender Bildgebung, umfassend:
ein Sichtfeld-Stitching-System nach einem der Ansprüche 1 bis 9;
ein Bildgebungsmodul (14) zum Empfangen des Detektionslichts und Erzeugen eines Detektionsbildes basierend auf dem Detektionslicht; und
einen Steuerabschnitt, der elektrisch mit dem Lichtquellenmodul (11), der Vielzahl von Lichtmodulatoren (131) und dem Bildgebungsmodul (14) verbunden ist, wobei der Steuerabschnitt konfiguriert ist, um einen Betriebsprozess des Lichtquellenmoduls (11), der Vielzahl von Lichtmodulatoren (131) und des Bildgebungsmoduls (14) zu steuern und ein biologisches Merkmal der biologischen Probe basierend auf dem Detektionsbild zu identifizieren.

11. Die Vorrichtung zur Identifizierung biologischer Proben basierend auf superauflösender Bildgebung nach Anspruch 10, wobei jeder der Vielzahl von Lichtmodulatoren (131) eine Vielzahl von Mikrospiegeln in der effektiven Lichtmodulationsfläche (132) umfasst, jeder der Vielzahl von Mikrospiegeln einen Rotationswinkel α aufweist, die effektive Lichtmodulationsfläche (132) jedes der Vielzahl von Lichtmodulatoren (131) rechteckig mit einer Länge L und einer Breite W ist, eine Länge jedes der Vielzahl von Lichtmodulatoren (131) X ist, eine Breite jedes der Vielzahl von Lichtmodulatoren (131) Y ist, die Vielzahl von zweiten Austrittsflächen (123) die erste Austrittsfläche (122) umgibt und axialsymmetrisch um eine Achse in einer X-Richtung und eine weitere Achse in einer Y-Richtung ist, die X-Richtung parallel zu einer Längsrichtung der Vielzahl von Lichtmodulatoren (131) ist und die Y-Richtung parallel zu einer Breitenrichtung der Vielzahl von Lichtmodulatoren (131) ist, die Vielzahl von Lichtmodulatoren in einer Array-Anordnung mit einer Vielzahl von Reihen mit einer gleichen Anzahl von Lichtmodulatoren (131) konfiguriert ist, die einen Abstand ΔX zwischen jeweils zwei benachbarten Lichtmodulatoren entlang der X-Richtung aufweist; auf einer Ebene, die durch die X-Richtung und eine optische Achse des Lichtführungselements (12) gebildet wird, ein Kegelwinkel zwischen der Eintrittsfläche (121) und der Vielzahl von zweiten Austrittsflächen (123) korrespondierend zu der Vielzahl von Lichtmodulatoren, die entlang der X-Richtung angeordnet sind, α ist; ein Brechungsindex des Lichtführungselements (12) n ist, ein Abstand zwischen der ersten Austrittsfläche (122) und dem Lichtmodulationsmodul (13) entlang der optischen Achse H ist, und die folgende Bedingung erfüllt ist: $2 Htan \left[α\left(n-1\right)\right] = 2 X + 2 ΔX + L .$

12. Die Vorrichtung zur Identifizierung biologischer Proben basierend auf superauflösender Bildgebung nach Anspruch 11, wobei ein Abstand zwischen benachbarten Lichtmodulatoren (131) entlang der Y-Richtung ΔY ist;
auf einer Ebene, die durch die optische Achse und die Y-Richtung gebildet wird, ein Kegelwinkel zwischen der Vielzahl von zweiten Austrittsflächen (123) und der Eintrittsfläche (121) korrespondierend zu der Vielzahl von Lichtmodulatoren (131) entlang der Y-Richtung β ist, und die folgende Bedingung erfüllt ist: $2 Htan \left[β\left(n-1\right)\right] = 2 Y + 2 ΔY + W .$

13. Ein Sichtfeld-Stitching-Verfahren, das auf ein Sichtfeld-Stitching-System angewendet wird, wobei das Sichtfeld-Stitching-System ein Lichtquellenmodul (11), ein Lichtführungselement (12) und ein Lichtmodulationsmodul (13) aufweist, wobei das Lichtmodulationsmodul (13) mit einer Vielzahl von Lichtmodulatoren (131) versehen ist, das Lichtführungselement (12) mit einer Eintrittsfläche (121), einer der Eintrittsfläche (121) zugewandten ersten Austrittsfläche (122) und einer Vielzahl von zweiten Austrittsflächen (123), die sich biegen und von Rändern der ersten Austrittsfläche (122) in Richtung der Eintrittsfläche (121) erstrecken, versehen ist, eine Anzahl der Vielzahl von Lichtmodulatoren (131) gleich einer Gesamtzahl der ersten Austrittsfläche (122) und der Vielzahl von zweiten Austrittsflächen (123) ist, und jeder der Vielzahl von Lichtmodulatoren (131) eine effektive Lichtmodulationsfläche (132) aufweist; wobei das Sichtfeld-Stitching-Verfahren umfasst:
emittieren eines ersten Strahls (L1) in Richtung der Eintrittsfläche (121) durch das Lichtquellenmodul (11);
aufteilen des ersten Strahls (L1) in eine Vielzahl von zweiten Strahlen (L2) durch die erste Austrittsfläche (122) und die Vielzahl von zweiten Austrittsflächen (123), und ferner Lenken der Vielzahl von zweiten Strahlen (L2), sodass sie aus der ersten Austrittsfläche (122) und der Vielzahl von zweiten Austrittsflächen (123) austreten und sich entlang eines optischen Pfads des ersten Strahls (L1) ausbreiten;
übertragen jedes der Vielzahl von zweiten Strahlen (L2) zu einem der Vielzahl von Lichtmodulatoren (131), Steuern jedes der Vielzahl von Lichtmodulatoren (131), um einen der Vielzahl von zweiten Strahlen (L2) zu empfangen und regulär zu reflektieren, und wobei jeder der Vielzahl von zweiten Strahlen (L2) die effektive Lichtmodulationsfläche (132) des Lichtmodulators (131) abdeckt; und
bilden eines Beleuchtungslichts (L3) auf der Eintrittsfläche (121) durch eine Kombination der Vielzahl von zweiten Strahlen (L2), die entlang einer entgegengesetzten Richtung des optischen Pfads regulär zum Lichtführungselement (12) reflektiert werden und durch die erste Austrittsfläche (122) und die Vielzahl von zweiten Austrittsflächen (123) hindurchtreten.

14. Das Sichtfeld-Stitching-Verfahren nach Anspruch 13, wobei jeder der Vielzahl von Lichtmodulatoren (131) mit einer Vielzahl von Mikrospiegeln in der effektiven Lichtmodulationsfläche (132) versehen ist, ein drehbarer Winkel jedes der Vielzahl von Mikrospiegeln α ist, die effektive Lichtmodulationsfläche (132) rechteckig mit einer Länge von L und einer Breite von W ist, jeder der Vielzahl von Lichtmodulatoren (131) eine Länge von X und eine Breite von Y aufweist, die Vielzahl von zweiten Austrittsflächen (123) symmetrisch um die erste Austrittsfläche (122) und um eine Achse in einer X-Richtung und eine weitere Achse in einer Y-Richtung verteilt ist, die X-Richtung parallel zu einer Längsrichtung der Vielzahl von Lichtmodulatoren (131) ist, die Y-Richtung parallel zu einer Breitenrichtung der Vielzahl von Lichtmodulatoren (131) ist, die Vielzahl von Lichtmodulatoren (131) in einer symmetrischen Anordnung entlang der X-Richtung und der Y-Richtung angeordnet ist; wobei auf einer Ebene, die durch eine optische Achse des Lichtführungselements (12) und die X-Richtung gebildet wird, das Sichtfeld-Stitching-Verfahren ferner umfasst:
einstellen eines Abstands zwischen jeweils zwei benachbarten Lichtmodulatoren (131), um das Beleuchtungslicht (L3) zu erzeugen, umfassend: Einstellen des Abstands zwischen jeweils zwei benachbarten Lichtmodulatoren (131) entlang der X-Richtung, und Einstellen einer Distanz H zwischen der ersten Austrittsfläche (122) und dem Lichtmodulationsmodul (13) entlang der optischen Achse; wobei der Abstand zwischen jeweils zwei benachbarten Lichtmodulatoren (131) entlang der X-Richtung ΔX ist, ein Kegelwinkel zwischen der Eintrittsfläche und der Vielzahl von zweiten Austrittsflächen korrespondierend zu der Vielzahl von Lichtmodulatoren, die entlang der X-Richtung angeordnet sind,
α ist, der Kegelwinkel α gleich dem Rotationswinkel α ist und eine Bedingung von $2 Htan \left[α\left(n-1\right)\right] = 2 X + 2 ΔX + L erfüllt$ ist;
und/oder
einstellen des Abstands zwischen jeweils zwei benachbarten Lichtmodulatoren (131) entlang der Y-Richtung gemäß einer Bedingung von $2 Htan \left[β\left(n-1\right)\right] = 2 Y + 2 ΔY + W ,$
wobei der Abstand zwischen jeweils zwei benachbarten Lichtmodulatoren (131) entlang der Y-Richtung ΔY ist; und auf einer Ebene, die durch eine optische Achse und die Y-Richtung gebildet wird, ein Kegelwinkel zwischen der Eintrittsfläche (121) und der Vielzahl von zweiten Austrittsflächen (123) korrespondierend zu der Vielzahl von Lichtmodulatoren (131), die entlang der Y-Richtung angeordnet sind, β ist.

15. Ein Verfahren zur Identifizierung biologischer Proben basierend auf superauflösender Bildgebung, das auf eine Vorrichtung zur Identifizierung biologischer Proben angewendet wird, wobei die Vorrichtung zur Identifizierung biologischer Proben mit einem Sichtfeld-Stitching-System versehen ist, das ein Lichtquellenmodul (11), ein Lichtführungselement (12) und ein Lichtmodulationsmodul (13) umfasst, wobei das Lichtmodulationsmodul (13) mit einer Vielzahl von Lichtmodulatoren (131) versehen ist, das Lichtführungselement (12) mit einer Eintrittsfläche (121), die korrespondierend zu dem Lichtquellenmodul (11) positioniert ist, einer ersten Austrittsfläche (122), die der Eintrittsfläche (121) zugewandt und von dieser beabstandet positioniert ist, und einer Vielzahl von zweiten Austrittsflächen (123), die sich biegen und von Rändern der ersten Austrittsfläche (122) in Richtung der Eintrittsfläche (121) erstrecken, versehen ist, eine Anzahl der Vielzahl von Lichtmodulatoren (131) gleich einer Gesamtzahl der ersten Austrittsfläche (122) und der Vielzahl von zweiten Austrittsflächen (123) ist, und jeder der Vielzahl von Lichtmodulatoren (131) eine effektive Lichtmodulationsfläche (132) aufweist; wobei das Verfahren zur Identifizierung biologischer Proben basierend auf superauflösender Bildgebung umfasst:
ein Sichtfeld-Stitching-Verfahren nach Anspruch 13 oder 14;
bestrahlen einer biologischen Probe mit dem Beleuchtungslicht (L3) durch die Eintrittsfläche (121), um ein Detektionslicht von der biologischen Probe zu erzeugen;
sammeln des Detektionslichts und Erzeugen eines Detektionsbildes basierend auf dem Detektionslicht; und
identifizieren eines biologischen Merkmals der biologischen Probe basierend auf dem Detektionsbild.

16. Das Verfahren zur Identifizierung biologischer Proben basierend auf superauflösender Bildgebung nach Anspruch 15, wobei jeder der Vielzahl von Lichtmodulatoren (131) mit einer Vielzahl von Mikrospiegeln in der effektiven Lichtmodulationsfläche (132) versehen ist, ein drehbarer Winkel jedes der Vielzahl von Mikrospiegeln α ist, die effektive Lichtmodulationsfläche (132) rechteckig mit einer Länge von L und einer Breite von W ist, jeder der Vielzahl von Lichtmodulatoren (131) eine Länge von X und eine Breite von Y aufweist, die Vielzahl von zweiten Austrittsflächen (123) symmetrisch um die erste Austrittsfläche (122) und um eine Achse in einer X-Richtung und eine weitere Achse in einer Y-Richtung verteilt ist, die X-Richtung parallel zu einer Längsrichtung der Vielzahl von Lichtmodulatoren (131) ist, die Y-Richtung parallel zu einer Breitenrichtung der Vielzahl von Lichtmodulatoren (131) ist, die Vielzahl von Lichtmodulatoren (131) in einer symmetrischen Anordnung entlang der X-Richtung und der Y-Richtung angeordnet ist; wobei auf einer Ebene, die durch eine optische Achse des Lichtführungselements (12) und die X-Richtung gebildet wird, das Sichtfeld-Stitching-Verfahren ferner umfasst:
einstellen eines Abstands zwischen jeweils zwei benachbarten Lichtmodulatoren (131), um das Beleuchtungslicht (L3) zu erzeugen, umfassend: Einstellen des Abstands zwischen jeweils zwei benachbarten Lichtmodulatoren (131) entlang der X-Richtung, und Einstellen einer Distanz H zwischen der ersten Austrittsfläche (122) und dem Lichtmodulationsmodul (13) entlang der optischen Achse; wobei der Abstand zwischen jeweils zwei benachbarten Lichtmodulatoren (131) entlang der X-Richtung ΔX ist, ein Kegelwinkel zwischen der Eintrittsfläche (121) und der Vielzahl von zweiten Austrittsflächen (123) korrespondierend zu der Vielzahl von Lichtmodulatoren (131), die entlang der X-Richtung angeordnet sind, α ist, der Kegelwinkel α gleich dem Rotationswinkel α ist und eine Bedingung von $2 Htan \left[α\left(n-1\right)\right] = 2 X + 2 ΔX + L erfüllt$ ist;
und/oder
einstellen des Abstands zwischen jeweils zwei benachbarten Lichtmodulatoren (131) entlang der Y-Richtung gemäß einer Bedingung $2 Htan \left[β\left(n-1\right)\right] = 2 Y + 2 ΔY + W ,$
wobei der Abstand zwischen jeweils zwei benachbarten Lichtmodulatoren (131) entlang der Y-Richtung ΔY ist; und auf einer Ebene, die durch eine optische Achse und die Y-Richtung gebildet wird, ein Kegelwinkel zwischen der Eintrittsfläche (121) und der Vielzahl von zweiten Austrittsflächen (123) korrespondierend zu der Vielzahl von Lichtmodulatoren (131), die entlang der Y-Richtung angeordnet sind, β ist.

## Revendications

1. Un système de couture de champ de vision comprenant:
un module de source lumineuse (11) pour émettre un premier faisceau (L1);
un élément de guidage de lumière (12) sur un chemin optique du premier faisceau (L1) et configuré pour diviser le premier faisceau (L1) en une pluralité de seconds faisceaux (L2), dans lequel l'élément de guidage de lumière (12) comprend une surface incidente (121) positionnée de manière correspondante au module de source lumineuse (11), une première surface de sortie (122) faisant face à et espacée de la surface incidente (121), et une pluralité de secondes surfaces de sortie (123) se courbant et s'étendant depuis des bords de la première surface de sortie (122) vers la surface incidente (121), la pluralité de seconds faisceaux (L2) sortent de la première surface de sortie (122) et de la pluralité de secondes surfaces de sortie (123) et se propagent le long du chemin optique; et
un module de modulation de lumière (13) comprenant une pluralité de modulateurs de lumière (131), dans lequel un nombre de la pluralité de modulateurs de lumière (131) est le même qu'un nombre total de la première surface de sortie (122) et de la pluralité de secondes surfaces de sortie (123), chacun de la pluralité de modulateurs de lumière (131) est configuré pour recevoir et réfléchir régulièrement l'un de la pluralité de seconds faisceaux (L2), chacun de la pluralité de modulateurs de lumière (131) a une zone de modulation de lumière effective (132), chacun de la pluralité de seconds faisceaux (L2) couvre la zone de modulation de lumière effective (132) de l'un des modulateurs de lumière (131) lorsqu'il est incident sur le modulateur de lumière (131);
dans lequel l'élément de guidage de lumière (12) est en outre configuré pour recevoir et combiner la pluralité de seconds faisceaux (L2) réfléchis par la pluralité de modulateurs de lumière (131) afin de former une lumière d'éclairage (L3) pour sortir en outre à travers la surface incidente (121).

2. Le système de couture de champ de vision selon la revendication 1, dans lequel un faisceau de la pluralité de seconds faisceaux (L2) sort de la première surface de sortie (122), et d'autres seconds faisceaux (L2) de la pluralité de seconds faisceaux (L2) sortent de la pluralité de secondes surfaces de sortie (123), la première surface de sortie (122) est connectée à la pluralité de secondes surfaces de sortie (123), et la pluralité de secondes surfaces de sortie (123) entourent la première surface de sortie (122).

3. Le système de couture de champ de vision selon la revendication 2, dans lequel l'élément de guidage de lumière (12) comprend une partie d'étage (124) et une partie en saillie (125), la partie en saillie (125) est connectée à une extrémité de la partie d'étage (124) et est coaxiale avec la partie d'étage (124), une surface de la partie en saillie (125) comprend la première surface de sortie (122) et la pluralité de secondes surfaces de sortie (123), une surface de la partie d'étage (124) éloignée de la partie en saillie (125) est la surface incidente (121), et la surface incidente (121) est parallèle à la première surface de sortie (122).

4. Le système de couture de champ de vision selon la revendication 3, dans lequel la première surface de sortie (122) est polygonale;
un nombre de la pluralité de secondes surfaces de sortie (123) est égal à un nombre de côtés de la première surface de sortie (122); ou un nombre de la pluralité de secondes surfaces de sortie (123) est le double d'un nombre de côtés de la première surface de sortie (122), la seconde surface de sortie (123) comprend une pluralité de premières surfaces de réfraction et une pluralité de secondes surfaces de réfraction, avec une correspondance biunivoque entre la pluralité de premières surfaces de réfraction et les côtés de la première surface de sortie (122), et une correspondance biunivoque entre la pluralité de secondes surfaces de réfraction et les coins de la première surface de sortie (122), et chacune de la pluralité de secondes surfaces de réfraction est formée entre deux quelconques premières surfaces de réfraction adjacentes.

5. Le système de couture de champ de vision selon l'une quelconque des revendications 3 à 4, dans lequel la partie d'étage (124) a la forme d'un cylindre avec une pluralité d'arêtes, et un nombre de la pluralité d'arêtes est le même qu'un nombre de la pluralité de secondes surfaces de sortie (123).

6. Le système de couture de champ de vision selon l'une quelconque des revendications 1 à 4, dans lequel chacun de la pluralité de modulateurs de lumière (131) comprend une pluralité de micromiroirs dans la zone de modulation de lumière effective (132), chacun de la pluralité de micromiroirs a un angle de rotation α, la zone de modulation de lumière effective (132) de chacun de la pluralité de modulateurs de lumière (131) est rectangulaire avec une longueur L et une largeur W, une longueur de chacun de la pluralité de modulateurs de lumière (131) est X, une largeur de chacun de la pluralité de modulateurs de lumière (131) est Y, la pluralité de secondes surfaces de sortie (123) entourent la première surface de sortie (122) et sont à symétrie axiale autour d'un axe dans une direction X et d'un autre axe dans une direction Y, la direction X est parallèle à une direction de longueur de la pluralité de modulateurs de lumière (131), et la direction Y est parallèle à une direction de largeur de la pluralité de modulateurs de lumière (131), la pluralité de modulateurs de lumière (131) sont configurés dans un agencement en réseau avec une pluralité de rangées ayant un même nombre de modulateurs de lumière (131), ayant un espace ΔX entre chaque deux modulateurs de lumière adjacents le long de la direction X; sur un plan formé par la direction X et un axe optique de l'élément de guidage de lumière (12), un angle de cône entre la surface incidente (121) et la pluralité de secondes surfaces de sortie (123) correspondant à la pluralité de modulateurs de lumière (131) agencés le long de la direction X est α; un indice de réfraction de l'élément de guidage de lumière (12) est n, une distance entre la première surface de sortie (122) et le module de modulation de lumière (13) le long de l'axe optique est H, et la condition suivante est satisfaite: $2 Htan \left[α\left(n-1\right)\right] = 2 X + 2 ΔX + L .$

7. Le système de couture de champ de vision selon la revendication 6, dans lequel une distance entre des modulateurs de lumière (131) adjacents le long de la direction Y est ΔY;
sur un plan formé par l'axe optique et la direction Y, un angle de cône entre la pluralité de secondes surfaces de sortie (123) et la surface incidente (121) correspondant à la pluralité de modulateurs de lumière (131) le long de la direction Y est β, et la condition suivante est satisfaite: $2 Htan \left[β\left(n-1\right)\right] = 2 Y + 2 ΔY + W .$

8. Le système de couture de champ de vision selon l'une quelconque des revendications 1 à 4, dans lequel le module de source lumineuse (11) comprend au moins un dispositif laser (111) et au moins une lentille d'expansion de faisceau (112), l'au moins une lentille d'expansion de faisceau (112) est positionnée entre l'au moins un dispositif laser (111) et l'élément de guidage de lumière (12);
l'au moins un dispositif laser (111) est configuré pour émettre le premier faisceau (L1), l'au moins une lentille d'expansion de faisceau (112) est configurée pour recevoir, étendre et collimater le premier faisceau (L1), et une zone de spot du premier faisceau (L1) après avoir été étendu et collimaté par l'au moins une lentille d'expansion de faisceau (112) est une somme de la zone de modulation de lumière effective (132) de la pluralité de modulateurs de lumière (131).

9. Le système de couture de champ de vision selon l'une quelconque des revendications 1 à 4, dans lequel une zone de champ de vision de la lumière d'éclairage (L3) est supérieure à une zone de champ de vision de l'un quelconque de la pluralité de seconds faisceaux (L2).

10. Un dispositif d'identification d'échantillon biologique (10) basé sur l'imagerie à super-résolution, comprenant:
un système de couture de champ de vision selon l'une quelconque des revendications 1 à 9;
un module d'imagerie (14) pour recevoir la lumière de détection et générer une image de détection basée sur la lumière de détection; et
une partie de commande connectée électriquement au module de source lumineuse (11), à la pluralité de modulateurs de lumière (131) et au module d'imagerie (14), la partie de commande est configurée pour commander un processus de fonctionnement du module de source lumineuse (11), de la pluralité de modulateurs de lumière (131) et du module d'imagerie (14), et pour identifier une caractéristique biologique de l'échantillon biologique sur la base de l'image de détection.

11. Le dispositif d'identification d'échantillon biologique basé sur l'imagerie à super-résolution selon la revendication 10, dans lequel chacun de la pluralité de modulateurs de lumière (131) comprend une pluralité de micromiroirs dans la zone de modulation de lumière effective (132), chacun de la pluralité de micromiroirs a un angle de rotation α, la zone de modulation de lumière effective (132) de chacun de la pluralité de modulateurs de lumière (131) est rectangulaire avec une longueur L et une largeur W, une longueur de chacun de la pluralité de modulateurs de lumière (131) est X, une largeur de chacun de la pluralité de modulateurs de lumière (131) est Y, la pluralité de secondes surfaces de sortie (123) entourent la première surface de sortie (122) et sont à symétrie axiale autour d'un axe dans une direction X et d'un autre axe dans une direction Y, la direction X est parallèle à une direction de longueur de la pluralité de modulateurs de lumière (131), et la direction Y est parallèle à une direction de largeur de la pluralité de modulateurs de lumière (131), la pluralité de modulateurs de lumière sont configurés dans un agencement en réseau avec une pluralité de rangées ayant un même nombre de modulateurs de lumière (131), ayant un espace ΔX entre chaque deux modulateurs de lumière adjacents le long de la direction X; sur un plan formé par la direction X et un axe optique de l'élément de guidage de lumière (12), un angle de cône entre la surface incidente (121) et la pluralité de secondes surfaces de sortie (123) correspondant à la pluralité de modulateurs de lumière agencés le long de la direction X est α; un indice de réfraction de l'élément de guidage de lumière (12) est n, une distance entre la première surface de sortie (122) et le module de modulation de lumière (13) le long de l'axe optique est H, et la condition suivante est satisfaite: $2 Htan \left[α\left(n-1\right)\right] = 2 X + 2 ΔX + L .$

12. Le dispositif d'identification d'échantillon biologique basé sur l'imagerie à super-résolution selon la revendication 11, dans lequel une distance entre des modulateurs de lumière (131) adjacents le long de la direction Y est ΔY;
sur un plan formé par l'axe optique et la direction Y, un angle de cône entre la pluralité de secondes surfaces de sortie (123) et la surface incidente (121) correspondant à la pluralité de modulateurs de lumière (131) le long de la direction Y est β, et la condition suivante est satisfaite: $2 Htan \left[β\left(n-1\right)\right] = 2 Y + 2 ΔY + W .$

13. Un procédé de couture de champ de vision appliqué à un système de couture de champ de vision, dans lequel le système de couture de champ de vision comprend un module de source lumineuse (11), un élément de guidage de lumière (12) et un module de modulation de lumière (13), le module de modulation de lumière (13) est pourvu d'une pluralité de modulateurs de lumière (131), l'élément de guidage de lumière (12) est pourvu d'une surface incidente (121), d'une première surface de sortie (122) faisant face à la surface incidente (121), et d'une pluralité de secondes surfaces de sortie (123) se courbant et s'étendant depuis des bords de la première surface de sortie (122) vers la surface incidente (121), un nombre de la pluralité de modulateurs de lumière (131) est le même qu'un nombre total de la première surface de sortie (122) et de la pluralité de secondes surfaces de sortie (123), et chacun de la pluralité de modulateurs de lumière (131) a une zone de modulation de lumière effective (132); le procédé de couture de champ de vision comprend:
émettre un premier faisceau (L1) vers la surface incidente (121) par le module de source lumineuse (11);
diviser le premier faisceau (L1) en une pluralité de seconds faisceaux (L2) par la première surface de sortie (122) et la pluralité de secondes surfaces de sortie (123), et en outre diriger la pluralité de seconds faisceaux (L2) pour qu'ils sortent de la première surface de sortie (122) et de la pluralité de secondes surfaces de sortie (123) et se propagent le long d'un chemin optique du premier faisceau (L1); transmettre chacun de la pluralité de seconds faisceaux (L2) à l'un de la pluralité de modulateurs de lumière (131), commander chacun de la pluralité de modulateurs de lumière (131) pour recevoir et réfléchir régulièrement l'un de la pluralité de seconds faisceaux (L2), et avec chacun de la pluralité de seconds faisceaux (L2) couvrant la zone de modulation de lumière effective (132) du modulateur de lumière (131); et
former une lumière d'éclairage (L3) sur la surface incidente (121) par une combinaison de la pluralité de seconds faisceaux (L2) qui sont réfléchis régulièrement vers l'élément de guidage de lumière (12) le long d'une direction opposée du chemin optique et passent à travers la première surface de sortie (122) et la pluralité de secondes surfaces de sortie (123).

14. Le procédé de couture de champ de vision selon la revendication 13, dans lequel chacun de la pluralité de modulateurs de lumière (131) est pourvu d'une pluralité de micromiroirs dans la zone de modulation de lumière effective (132), un angle de rotation de chacun de la pluralité de micromiroirs est α, la zone de modulation de lumière effective (132) est rectangulaire avec une longueur de L et une largeur de W, chacun de la pluralité de modulateurs de lumière (131) a une longueur de X et une largeur de Y, la pluralité de secondes surfaces de sortie (123) sont réparties symétriquement autour de la première surface de sortie (122) et autour d'un axe dans une direction X et d'un autre axe dans une direction Y, la direction X est parallèle à une direction de longueur de la pluralité de modulateurs de lumière (131), la direction Y est parallèle à une direction de largeur de la pluralité de modulateurs de lumière (131), la pluralité de modulateurs de lumière (131) sont agencés selon un réseau symétrique le long de la direction X et de la direction Y; sur un plan formé par un axe optique de l'élément de guidage de lumière (12) et la direction X, le procédé de couture de champ de vision comprend en outre:
ajuster l'espace entre chaque deux modulateurs de lumière (131) adjacents pour générer la lumière d'éclairage (L3), comprenant: ajuster l'espace entre chaque deux modulateurs de lumière (131) adjacents le long de la direction X, et ajuster une distance H entre la première surface de sortie (122) et le module de modulation de lumière (13) le long de l'axe optique; l'espace entre chaque deux modulateurs de lumière (131) adjacents le long de la direction X est ΔX, un angle de cône entre la surface incidente et la pluralité de secondes surfaces de sortie correspondant à la pluralité de modulateurs de lumière agencés le long de la direction X est α, l'angle de cône α est égal à l'angle de rotation α, et une condition de $2 Htan \left[α\left(n-1\right)\right] = 2 X + 2 ΔX + L$ est satisfaite;
et/ou
ajuster l'espace entre chaque deux modulateurs de lumière (131) adjacents le long de la direction Y selon une condition de $2 Htan \left[β\left(n-1\right)\right] = 2 Y + 2 ΔY + W ,$
dans lequel l'espace entre chaque deux modulateurs de lumière (131) adjacents le long de la direction Y est ΔY; et sur un plan formé par un axe optique et la direction Y, un angle de cône entre la surface incidente (121) et la pluralité de secondes surfaces de sortie (123) correspondant à la pluralité de modulateurs de lumière (131) agencés le long de la direction Y est β.

15. Un procédé d'identification d'échantillon biologique basé sur l'imagerie à super-résolution et qui est appliqué à un dispositif d'identification d'échantillon biologique, le dispositif d'identification d'échantillon biologique est pourvu d'un système de couture de champ de vision comprenant un module de source lumineuse (11), un élément de guidage de lumière (12) et un module de modulation de lumière (13), le module de modulation de lumière (13) est pourvu d'une pluralité de modulateurs de lumière (131), l'élément de guidage de lumière (12) est pourvu d'une surface incidente (121) positionnée de manière correspondante au module de source lumineuse (11), d'une première surface de sortie (122) faisant face à et espacée de la surface incidente (121), et d'une pluralité de secondes surfaces de sortie (123) se courbant et s'étendant depuis des bords de la première surface de sortie (122) vers la surface incidente (121), un nombre de la pluralité de modulateurs de lumière (131) est le même qu'un nombre total de la première surface de sortie (122) et de la pluralité de secondes surfaces de sortie (123), et chacun de la pluralité de modulateurs de lumière (131) a une zone de modulation de lumière effective (132); le procédé d'identification d'échantillon biologique basé sur l'imagerie à super-résolution comprend:
un procédé de couture de champ de vision selon la revendication 13 ou 14;
irradier un échantillon biologique avec la lumière d'éclairage (L3) à travers la surface incidente (121) pour générer une lumière de détection à partir de l'échantillon biologique;
collecter la lumière de détection et générer une image de détection basée sur la lumière de détection; et
identifier une caractéristique biologique de l'échantillon biologique sur la base de l'image de détection.

16. Le procédé d'identification d'échantillon biologique basé sur l'imagerie à super-résolution selon la revendication 15, dans lequel chacun de la pluralité de modulateurs de lumière (131) est pourvu d'une pluralité de micromiroirs dans la zone de modulation de lumière effective (132), un angle de rotation de chacun de la pluralité de micromiroirs est α, la zone de modulation de lumière effective (132) est rectangulaire avec une longueur de L et une largeur de W, chacun de la pluralité de modulateurs de lumière (131) a une longueur de X et une largeur de Y, la pluralité de secondes surfaces de sortie (123) sont réparties symétriquement autour de la première surface de sortie (122) et autour d'un axe dans une direction X et d'un autre axe dans une direction Y, la direction X est parallèle à une direction de longueur de la pluralité de modulateurs de lumière (131), la direction Y est parallèle à une direction de largeur de la pluralité de modulateurs de lumière (131), la pluralité de modulateurs de lumière (131) sont agencés selon un réseau symétrique le long de la direction X et de la direction Y; sur un plan formé par un axe optique de l'élément de guidage de lumière (12) et la direction X, le procédé de couture de champ de vision comprend en outre:
ajuster l'espace entre chaque deux modulateurs de lumière (131) adjacents pour générer la lumière d'éclairage (L3), comprenant: ajuster l'espace entre chaque deux modulateurs de lumière (131) adjacents le long de la direction X, et ajuster une distance H entre la première surface de sortie (122) et le module de modulation de lumière (13) le long de l'axe optique; dans lequel l'espace entre des modulateurs de lumière (131) adjacents le long de la direction X est ΔX, un angle de cône entre la surface incidente (121) et la pluralité de secondes surfaces de sortie (123) correspondant à la pluralité de modulateurs de lumière (131) agencés le long de la direction X est α, l'angle de cône α est égal à l'angle de rotation α, et une condition de $2 Htan \left[α\left(n-1\right)\right] = 2 X + 2 ΔX + L$ est satisfaite;
et/ou
ajuster l'espace entre chaque deux modulateurs de lumière (131) adjacents le long de la direction Y selon une condition $2 Htan \left[β\left(n-1\right)\right] = 2 Y + 2 ΔY + W ,$
dans lequel l'espace entre chaque deux modulateurs de lumière (131) adjacents le long de la direction Y est ΔY; et sur un plan formé par un axe optique et la direction Y, un angle de cône entre la surface incidente (121) et la pluralité de secondes surfaces de sortie (123) correspondant à la pluralité de modulateurs de lumière (131) agencés le long de la direction Y est β.
